# FASCICULE DE BREVET EUROPEEN

(11) **EP 3 724 157 B1**
(45) Date de publication et mention de la délivrance du brevet: **17.11.2021**
(21) Numéro de dépôt: 18808021.2
(22) Date de dépôt: 30.11.2018
(51) Int. Cl.: C07C 7/13, C07C 7/11, C07C 11/08, C07C 11/09

(54) **PROCEDE D'ELIMINATION SIMULTANEE D'ISOBUTANAL ET D'ACETONE DE CHARGES OLEFINIQUES PAR ADSORPTION SUR UN MATERIAU ZEOLITHIQUE**
VERFAHREN ZUR GLEICHZEITIGEN BESEITIGUNG VON ISOBUTANAL UND ACETON AUS OLEFINISCHEN AUSGANGSMATERIALIEN DURCH ADSORPTION AN EINEM ZEOLITHMATERIAL
METHOD FOR SIMULTANEOUSLY ELIMINATING ISOBUTANAL AND ACETONE FROM OLEFINIC FEEDSTOCKS BY ADSORPTION ON A ZEOLITE MATERIAL

(30) Priorité: 13.12.2017 FR 1762090
(43) Date de publication de la demande: 21.10.2020
(73) Titulaire: IFP Energies nouvelles, 92852 Rueil-Malmaison (FR); Total Research & Technology Feluy, 7181 Seneffe (BE)
(72) Inventeur: BARTHELET, Karin, 69007 LYON (FR); BRACCO, Emmanuelle, 69420 CONDRIEU (FR); COUPARD, Vincent, 69100 VILLEURBANNE (FR); NESTERENKO, Nikolai, 1400 NIVELLES (BE)
(74) Mandataire: IFP Energies nouvelles
(86) Numéro de dépôt international: PCT/EP2018/083124
(87) Numéro de publication internationale: WO 2019/115251

(56) Documents cités:
- EP-A1- 2 547 639
- US-A1- 2013 245 348

## Description

### Domaine de l'invention

La présente invention concerne un procédé de purification d'une charge oléfinique comprenant une étape d'élimination simultanée d'aldéhydes et de cétones d'une charge oléfinique, opéré à basse température (inférieure ou égale à 200°C) par adsorption sur un adsorbant comprenant un matériau à charpente zéolithique. Plus particulièrement, l'invention concerne un procédé de purification d'une charge oléfinique comprenant des oléfines à 4 atomes de carbone et des impuretés comprenant l'isobutanal, l'éthanol et l'acétone, ledit procédé comprenant une étape d'élimination simultanée d'isobutanal et d'acétone par passage de ladite charge oléfinique, préalablement traitée, sur un lit fixe comprenant au moins un matériau à charpente zéolithique. Le pré-traitement de la charge oléfinique comprend au moins l'élimination de l'éthanol et de l'eau, contenus dans la charge oléfinique.

L'invention s'applique avantageusement au traitement des effluents de conversion de composés oxygénés et notamment au traitement de l'effluent issu de la déshydratation d'un isomère du butanol, notamment de l'isobutanol, ou d'un mélange d'isomères du butanol comprenant de l'isobutanol.

### Etat de la Technique

Les butènes produits par déshydratation d'alcool, en particulier par déshydratation d'un isomère du butanol, notamment de l'isobutanol, ou d'un mélange d'isomères du butanol comprenant de l'isobutanol, contiennent généralement quelques centaines de ppm massiques d'isobutanal, d'éthanol et d'acétone. Or pour respecter les spécifications imposées aux butènes, il s'avère nécessaire de les éliminer. En effet, les butènes produits peuvent être destinés à être envoyés dans une unité de métathèse qui utilise un catalyseur qui ne tolère pas plus de 10 ppm poids en oxygène. On entend par ppm poids en oxygène ou teneur en oxygène, la teneur massique en atome d'oxygène contenu dans le mélange considéré. Cette teneur en oxygène peut être calculée à partir de la composition massique ou molaire en composés hydrocarbonés oxygénés du mélange considéré.

Le brevet EP 2 547 639 B1, qui décrit un procédé de métathèse d'alcènes, explique ainsi que de nombreux polluants présents dans la charge de métathèse sont responsables de la chute d'activité du catalyseur.

Pour atteindre des niveaux de concentration en impuretés très faibles, une des techniques souvent utilisée est l'adsorption sur un solide mis en œuvre dans un lit fixe. Il s'agit néanmoins de trouver le ou les bons adsorbants pour permettre l'élimination de toutes les impuretés présentes.

Le brevet EP 2 547 639 B1 cite les alumines et zéolithes comme adsorbants potentiels. Cependant, aucun lien entre la nature des composés à éliminer et le choix de l'adsorbant pour obtenir un traitement efficace des alcènes n'est donné.

L'article C. C. Brunchi et. al., Ind. Eng. Chem. Res., 2012, 51, 16697-16708, décrit la co-adsorption en batch d'un mélange de composés organiques volatils (VOC), le butanal, le 2-éthyl-2-hexanal, le 2,6-diméthylcyclohexanone, le 2,4,6-triméthylphénol et le 2,4,6-triméthylanisole, dans du toluène. Les résultats obtenus montrent que la zéolithe NaY permet d'adsorber le butanal et le 2,6-diméthylcyclohexanone. Mais la bonne capacité d'adsorption par la NaY du butanal présent dans une charge aromatique tel que le toluène ne permet pas de déduire que la NaY pourrait éliminer l'isobutanal d'une charge oléfinique. En effet, il n'est pas évident de prédire la capacité et la sélectivité d'adsorption de molécules différentes présentes dans des solvants différents.

Le document Rouquerol J. et al.; « Adsorption by Powders & Porous Solids: Principle, methodology and applications (second édition) », Chapter 12. « Adsorption by clays, pillared clays, zeolites and aluminophosphates », 467-527, Academic Press, 2014, enseigne en effet que le mécanisme de sorption des molécules par les zéolithes en général et par la NaY en particulier est celui d'une adsorption physique, ou physisorption. Le phénomène de physisorption peut être défini comme la fixation de molécules d'adsorbat sur la surface d'un adsorbant par la mise en œuvre d'interactions faibles de type Van der Waals et/ou électrostatiques de polarisation, dipôle et quadrupôle pour les adsorbants ayant une structure ionique. Ces interactions ne sont pas spécifiques. De fait, il n'est pas évident de prédire la capacité et encore moins la sélectivité d'adsorption de molécules différentes présentes dans des solvants différents.

La faible adsorption par la NaY du 2-éthyl-2-hexanal comparativement au butanal (deux fois plus faible), montrée par C. C. Brunchi *et. al.,* illustre très bien cette non-évidence de la sélectivité d'adsorption par une zéolithe de différentes molécules de même famille chimique.

Le brevet US2013/245348A1 décrit un procédé de purification d'un courant d'isobutène contenant des impuretés telles que l'acétone, l'éthanol et des aldéhydes comme l'acétaldéhyde. La charge est mise en contact avec un lit fixe d'adsorbant zéolithe (pref. A, L, X, Y) optionnellement avec des cations compensateurs de charge. La charge peut être préalablement séchée.

La Demanderesse a mis en évidence que la mise en œuvre en lit fixe d'au moins un adsorbant comprenant au moins un matériau à charpente zéolithique permet d'éliminer simultanément l'isobutanal et l'acétone de charges oléfiniques comprenant des oléfines à 4 atomes de carbone, desquelles l'éthanol et l'eau ont été préalablement éliminés.

La présente invention concerne ainsi un procédé de purification d'une charge oléfinique comprenant des oléfines à 4 atomes de carbone, en particulier issue de la déshydratation d'un isomère du butanol, notamment de l'isobutanol, ou d'un mélange d'isomères du butanol comprenant de l'isobutanol, permettant l'élimination des impuretés isobutanal, acétone et éthanol, contenues à hauteur de quelques centaines de ppm massique dans ladite charge.

### Résumé de l'invention.

En particulier, la présente invention concerne un procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant une étape d'élimination de l'éthanol et de l'eau ; et
b) une étape d'élimination simultanée d'isobutanal et d'acétone, par passage de la charge pré-traitée issue de l'étape a) sur au moins un lit fixe d'au moins un adsorbant comprenant au moins un matériau à charpente zéolithique,
   ledit matériau à charpente zéolithique étant choisi dans le groupe constitué par: les zéolithes, AIPOs, SAPOs et leurs mélanges ;
   ledit matériau à charpente zéolithique possédant au moins des cages ou des canaux dont au moins une ouverture est définie par un anneau à au moins 10 atomes d'oxygène (10MR); ladite étape b) opérant à une température comprise entre 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

L'invention s'applique avantageusement au traitement des effluents de conversion de composés oxygénés et notamment au traitement de l'effluent issu de la déshydratation d'un isomère du butanol, notamment de l'isobutanol, ou d'un mélange d'isomères du butanol, comprenant de l'isobutanol. En particulier, l'invention s'applique au traitement de l'effluent issu de la déshydratation de l'isobutanol seul ou en mélange avec d'autres isomères du butanol.

Avantageusement, l'utilisation d'un lit fixe d'au moins un adsorbant spécifique, ledit adsorbant comprenant au moins un matériau à charpente zéolithique, dans les conditions particulières selon l'invention, permet d'éliminer les impuretés, notamment l'isobutanal et l'acétone, contenus dans le mélange de butènes issus de la déshydratation de l'isobutanol ou d'un mélange d'isomères du butanol comprenant de l'isobutanol.

Le procédé de purification selon l'invention permet ainsi d'obtenir, grâce à l'utilisation d'un lit fixe d'adsorbant spécifique et à des conditions optimisées, une teneur en oxygène, indicatrice de la teneur massique en impuretés hydrocarbonées oxygénées, dans le mélange d'oléfines à l'issue de la purification, inférieure à 50 ppm massique, préférentiellement inférieure ou égale à 30 ppm massique, de préférence inférieure ou égale à 15 ppm massique, de manière préférée inférieure ou égale à 10 ppm massique et de manière très préférée inférieure ou égale à 1 ppm massique. On entend par ppm poids en oxygène ou teneur en oxygène, la teneur massique en atome d'oxygène contenu dans le mélange considéré. Cette teneur en oxygène peut être calculée à partir de la composition massique ou molaire du mélange en composés hydrocarbonées oxygénés considéré. Avantageusement, le procédé selon l'invention permet d'obtenir des teneurs massiques en impuretés hydrocarbonées oxygénées, telles que l'isobutanal, l'éthanol et l'acétone, dans le mélange d'oléfines à l'issue de la purification, inférieures à 50 ppm, de préférence inférieures ou égales à 30 ppm, préférentiellement inférieures ou égales à 15 ppm, de manière préférée inférieures ou égales à 10 ppm et de manière très préférée inférieures ou égales à 1 ppm.

A la différence des adsorbants aluminiques, l'adsorbant comprenant au moins un matériau à charpente zéolithique peut réaliser le traitement de charges oléfiniques contenant de l'isobutanal et de l'acétone sans que cette dernière ne réagisse de manière indésirée avec l'isobutanal présent.

Dans le procédé selon l'invention, l'adsorbant comprenant un matériau à charpente zéolithique peut subir plusieurs cycles de régénération et conserver des capacités de captation importantes, ce qui peut présenter un réel avantage économique.

### Description de l'invention

Dans la présente invention, on entend par « teneur en impuretés hydrocarbonées oxygénées » ou « teneur massique en impuretés hydrocarbonées oxygénées » d'une charge ou d'un effluent (charge initiale, charge oléfinique ou effluent en sortie de procédé) la masse d'impuretés hydrocarbonées oxygénées, telles que l'isobutanal, l'éthanol et l'acétone, par unité de masse de la charge ou de l'effluent considéré. On citera par exemple comme méthode usuelle de détermination de la teneur en chacune des impuretés hydrocarbonées oxygénées la méthode UOP 960 qui utilise la chromatographie en phase gazeuse.

Il faut noter que l'oxygène de l'eau présente sous forme dissoute dans la charge ou l'effluent n'est pas comptabilisé dans la teneur en impuretés hydrocarbonées oxygénées. La teneur en eau présente sous forme dissoute dans la charge ou l'effluent peut être déterminée par une technique spécifique, par exemple selon la méthode Karl Fischer (cf. Analyse des solvants résiduels dans les produits pharmaceutiques, techniques de l'ingénieur P3260v1 M.Bauer 10/09/2001).

Dans la présente invention, on entend par « vitesse volumique horaire (VVH) de la charge sur le lit fixe » le ratio entre le débit volumique horaire de la charge à traiter et le volume du réacteur.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision serait apportée par la présente invention.

La présente invention consiste en un procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant une étape d'élimination de l'éthanol et de l'eau ; et
b) une étape d'élimination simultanée de l'isobutanal et de l'acétone, par passage de la charge pré-traitée issue de l'étape a) sur au moins un lit fixe d'au moins un adsorbant comprenant au moins un matériau à charpente zéolithique,
   ledit matériau à charpente zéolithique étant choisi dans le groupe constitué par: les zéolithes, AIPOs, SAPOs et leurs mélanges ;
   ledit matériau à charpente zéolithique possédant au moins des cages ou des canaux dont au moins une ouverture est définie par un anneau à au moins 10 atomes d'oxygène (10MR);
   ladite étape b) opérant à une température comprise entre 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

### La charge oléfinique

La charge oléfinique traitée par le procédé de purification selon l'invention est un mélange d'oléfines. Ce mélange peut contenir des oléfines linéaires, ramifiées ou un mélanges d'oléfines linéaires et ramifiées.

La charge oléfinique est caractérisée par sa teneur très élevée en oléfines. Elle contient de préférence au moins 95% poids, préférentiellement au moins 97% poids de son poids sec en oléfines. Le titre élevé en oléfines donne à cette coupe une réactivité particulièrement élevée, autant dans les étapes de valorisation avales que dans les étapes de transformation ou purification qu'elle subit.

Les oléfines présentes dans la charge sont des composés principalement à 4 atomes de carbone, plus particulièrement des butènes, notamment un mélange de n-butènes et d'isobutène.

La charge traitée selon l'invention peut également contenir des composés non oléfiniques parmi lesquels peuvent se trouver, en plus de l'eau, des composés organiques hydrocarbonés (impuretés). Les composés organiques hydrocarbonés peuvent être des paraffines, des diènes et des composés organiques oxygénés parmi lesquels on peut citer les aldéhydes, les cétones, les alcools, les acétals, les éthers esters, les furanes, les acides carboxyliques.

Plus particulièrement, la charge traitée selon l'invention comprend des oléfines à 4 atomes de carbone et des impuretés hydrocarbonées oxygénées comprenant l'isobutanal, l'éthanol et l'acétone.

Selon une variante de l'invention, la charge oléfinique est issue de la conversion de composés oxygénés. De préférence, la charge oléfinique traitée selon l'invention est issue de la déshydratation de l'isobutanol ou d'un mélange d'isomères du butanol comprenant de l'isobutanol. Préférentiellement, la charge oléfinique traitée selon l'invention est issue de la déshydratation d'un isomère du butanol, en particulier l'isobutanol, ou d'un mélange d'isomères du butanol comprenant de l'isobutanol. Typiquement, les butènes produits par déshydratation de l'isobutanol ou d'un mélange d'isomères du butanol comprenant de l'isobutanol, contiennent quelques centaines de ppm d'isobutanal, d'éthanol et d'acétone.

### Etape a)

Conformément à l'invention, la charge oléfinique subit un pré-traitement comprenant au moins une étape d'élimination de l'éthanol et d'eau que pourrait contenir la charge oléfinique. Ce pré-traitement permet d'augmenter la sélectivité de l'adsorbant, utilisé à l'étape b) suivante et comprenant au moins un matériau à charpente zéolithique, vis-à-vis de l'isobutanal et de l'acétone. Le pré-traitement permet donc d'optimiser la capacité de captation de l'isobutanal et de l'acétone par l'adsorbant à l'étape b).

En effet, la Demanderesse a découvert que parmi les impuretés présentes dans la charge, certaines sont incompatibles avec l'utilisation d'un adsorbant à base de matériau comprenant une charpente zéolithique, comme la zéolithe NaY par exemple. En particulier, la présence d'éthanol et/ou d'eau dans la charge réduit notablement les performances de l'adsorbant comprenant un matériau à charpente zéolithique. Il convient ainsi d'éliminer ces impuretés de la charge oléfinique, préalablement au passage sur l'adsorbant comprenant au moins un matériau à charpente zéolithique.

L'éthanol et l'eau contenus dans la charge oléfinique sont éliminés par toute méthode connue de l'homme du métier. En particulier, l'éthanol peut être éliminé par distillation ou par lavage à l'eau et l'eau peut être éliminée par adsorption sur tamis moléculaire par exemple de type 3A. Par exemple, l'éthanol est éliminé par lavage à l'eau de la charge oléfinique, suivi d'un séchage, par exemple sur un tamis de type 3A, comme cela est réalisé classiquement dans les procédés d'éthérification décrits, par exemple, dans l'ouvrage « Les biocarburants, état des lieux, perspectives et enjeux du développement », D.Ballerini, figure 2.13 page 109.

L'élimination de l'eau peut être réalisée simultanément à l'élimination de l'éthanol. Elle peut aussi être réalisée précédemment ou successivement à l'élimination de l'éthanol.

Avantageusement, à l'issue du pré-traitement (étape a), la charge oléfinique contient moins de 10 ppm massique, de préférence moins de 1 ppm massique d'eau, et moins de 10 ppm massique, de préférence moins de 1 ppm massique d'éthanol. Et plus particulièrement, la charge oléfinique est dépourvue d'éthanol et d'eau.

### L'adsorbant à charpente zéolithique

Conformément à l'invention, le procédé de purification comprend le passage de la charge oléfinique dans un réacteur comprenant au moins un lit fixe d'au moins un adsorbant spécifique.

Selon l'invention, l'adsorbant comprend au moins un matériau à charpente zéolithique. L'utilisation de l'adsorbant à charpente zéolithique permet d'éviter la réaction non désirée de condensation de l'isobutanal avec l'acétone, contrairement à d'autres types d'adsorbants comme par exemple les adsorbants aluminiques.

Le matériau à charpente zéolithique selon l'invention comprend au moins un élément tétravalent X choisi parmi le silicium, l'étain, le titane et le germanium. L'élément tétravalent X présent dans le matériau à charpente zéolithique selon l'invention est préférentiellement choisi parmi le silicium et le germanium et, de manière encore plus préférée, ledit élément X est le silicium.

A la place de l'élément X tétravalent, le matériau à charpente zéolithique selon l'invention peut selon un mode particulier de l'invention comprendre un élément pentavalent, tel que le phosphore.

Le matériau à charpente zéolithique selon l'invention comprend en outre, et selon un mode préféré de réalisation dudit matériau à charpente zéolithique, au moins un élément trivalent T choisi parmi l'aluminium, le fer, le bore, le gallium et l'indium. De préférence, l'élément T est l'aluminium.

Selon l'invention, le matériau à charpente zéolithique est avantageusement choisi dans le groupe constitué par: les zéolithes, AIPOs, SAPOs et leurs mélanges.

Avantageusement, le matériau à charpente zéolithique possède au moins des cages ou des canaux dont l'ouverture est définie par un anneau à au moins 10 atomes d'oxygène (10MR), de préférence au moins 12 atomes d'oxygène (12MR). En particulier, le matériau à charpente zéolithique possède uniquement des cages ou canaux dont l'ouverture est définie par un anneau à au moins 10 atomes d'oxygène (10MR), de préférence au moins 12 atomes d'oxygène (12MR).

Dans un mode de réalisation selon l'invention, le matériau à charpente zéolithique est choisi dans le groupe constitué par: AIPO-4, AIPO-5, AIPO-8, AIPO-11, JDF-20, VPI-5, SAPO-11, SAPO-40 ou SAPO-37.

Dans un autre mode de réalisation selon l'invention, le matériau à charpente zéolithique est une zéolithe. De préférence, le matériau à charpente zéolithique possède un type structural choisi parmi les types FAU, BEA, MOR, MFI, FER, EMT, TON, SZR, LTL (conformément aux codes attribués par la Commission des Structures de l'Association Internationales des Zéolithes (IZA). cf. http://www.iza-structure.org/databases/). Préférentiellement, le matériau à charpente zéolithique est de type structural FAU, MFI, BEA, LTL ou MOR, par exemple type Y, X, ZSM-5, beta, L, Mordénite ou leurs mélanges. De manière encore plus préférée, le matériau à charpente zéolithique est de type FAU, notamment Y ou X.

Avantageusement, le matériau à charpente zéolithique possède un ou plusieurs types de cations compensateurs de charge dans ses cages ou canaux, ces cations compensateurs pouvant aider à la sélectivité de l'adsorbant. De préférence, le ou les cations compensateurs de charges sont choisis parmi les alcalins et les alcalino-terreux. De manière très préférée, le ou les cations compensateurs de charge sont des alcalins, notamment des ions sodium. La charge du matériau à charpente zéolithique n'est pas compensée par des protons.

Le matériau à charpente zéolithique présente avantageusement un volume microporeux, déterminé par application de la norme ASTM D4365, compris entre 0,01 et 0,5 cm³.g⁻¹, de préférence entre 0,05 et 0,45 cm³.g⁻¹.

La surface spécifique du matériau à charpente zéolithique selon l'invention, déterminée par la méthode B.E.T par application de la norme ASTM 4365, est avantageusement comprise entre 200 et 1000 m².g⁻¹, de préférence entre 250 et 800 m².g⁻¹, de manière plus préférée entre 300 et 700 m².g⁻¹.

De préférence, le matériau à charpente zéolithique est mis en forme, sous forme de grains qui peuvent être de différentes formes et dimensions, selon toute méthode connue de l'homme du métier.

Selon une variante de l'invention, le matériau zéolitique est mis en forme avec un liant. La teneur en liant est alors comprise entre 5 et 30% en poids, de manière préférée entre 10 et 25% poids du poids de l'adsorbant mis en forme. Le liant est, de préférence, choisi parmi tous liants connus de l'homme du métier ; il peut être choisi dans les familles des oxydes ou hydroxydes réfractaires simples ou mixtes, des argiles, des charbons actifs, des résines vinyliques réticulées. De préférence, le liant est une argile et, plus particulièrement, le liant est une argile zéolithisable, c'est-à-dire que, par un traitement adéquat, l'argile est transformée en zéolithe, identique ou différente de celle mise en forme, augmentant ainsi la capacité d'adsorption de l'adsorbant. La zéolithisation peut se faire, par exemple, par traitement à la soude de l'adsorbant mis en forme suivi d'une calcination.

Avantageusement, l'adsorbant est utilisé dans le procédé de purification selon l'invention sous la forme d'un empilement de particules élémentaires dissociées ou sous la forme d'un ou plusieurs monolithes multicanaux installés en série ou en parallèle.

Dans le cas où l'adsorbant est utilisé sous la forme d'un empilement de particules élémentaires dissociées, il se présente sous la forme de billes, de cylindres multilobés, de préférence avec un nombre de lobes compris entre 2 et 5 ou sous forme d'anneaux, de cylindres creux, d'anneaux creux, d'anneaux de Raschig, de cylindres creux dentelés, de cylindres creux crénelés, de roues de charrettes, de selles de Berl ou de cylindres à multiples trous, seuls ou en mélange.

Dans le cas où l'adsorbant est utilisé sous la forme d'un ou plusieurs monolithes multicanaux installés en série ou en parallèle, il se présente de préférence sous la forme d'un monolithe multicanaux de type nid d'abeilles, les canaux pouvant être de section carrée, hexagonale, circulaire ou ovale. La surface des canaux du monolithe peut être lisse, cannelée ou rugueuse afin de favoriser un contact intime du fluide à traiter avec la surface des canaux du monolithe. La densité de canaux peut préférentiellement varier de 5 cpsi à 400 cpsi (cpsi = canal per square inch) (respectivement de environ 0,8 à environ 62,0 canaux par cm²). Alternativement, le monolithe peut être constitué de mousse céramique. La densité de pores de la mousse céramique est comprise entre 10 ppi et 60 ppi, et de manière préférée entre 10 et 30 ppi (ppi = pores per inch) (respectivement comprise entre environ 3,9 et environ 23,6 pores par cm et de manière préférée entre environ 3,9 et environ 11,8 pores par cm).

La section du monolithe doit être égale à la section interne du réacteur contenant le lit d'adsorbants afin de forcer l'intégralité du flux à traiter, à circuler à l'intérieur des canaux du monolithe.

Avantageusement, le lit fixe peut comprendre des adsorbants présentant un ou plusieurs types de mise en forme. L'adsorbant comprenant le matériau à charpente zéolithique peut être utilisé seul ou en mélange avec un ou des autre(s) adsorbant(s).

Il est particulièrement avantageux de superposer des adsorbants différents, en particulier de nature et/ou de mise en forme différentes, dans au moins deux lits fixes différents de hauteur variable, les adsorbants ayant le plus faible taux de vide, le taux de vide étant défini comme le rapport de la différence entre le volume du réacteur et le volume de solide sur le volume de réacteur (c'est-à-dire présentant le plus faible volume rempli par l'adsorbant considéré, rapporté au volume du réacteur) étant de préférence utilisés dans le ou les premiers lits fixes, en entrée du réacteur afin de réaliser une sorte de filtration de la charge lors de son passage dans le ou les lits. Des adsorbant différents peuvent également être mélangés au sein d'un même lit.

Selon un mode de réalisation de l'invention, l'adsorbant peut être activé, notamment in situ, juste avant sa première utilisation dans le procédé selon l'invention, selon l'une quelconque des méthodes connues de l'Homme du métier. Avantageusement, l'activation est réalisée par passage d'un gaz chauffé entre 100 et 500°C. Le gaz utilisé peut être un gaz de combustion, de l'air, de l'azote. De préférence le gaz d'activation est l'azote.

### Etape b)

Conformément à l'invention, le procédé de purification de la charge oléfinique, en particulier l'étape d'élimination simultanée de l'isobutanal et de l'acétone, opère à une température comprise entre 0°C et 200°C, de préférence comprise entre 10 et 100°C et de préférence entre 20 et 60°C, par exemple à 30°C, à une pression comprise entre 0,1 et 10 MPa, de préférence comprise entre 0,3 et 5 MPa et avec une vitesse volumique horaire (VVH) de ladite charge sur ledit lit fixe comprise entre 0,1 et 10 h⁻¹ et de préférence comprise entre 0,2 et 5 h⁻¹.

Dans ces conditions et grâce à l'adsorbant spécifique utilisé dans le procédé de purification selon l'invention, l'adsorption des aldéhydes et des cétones, en particulier l'isobutanal et l'acétone, est observée à la surface de l'adsorbant à des capacités satisfaisantes.

Le procédé de purification selon l'invention, grâce notamment à l'adsorbant spécifique utilisé dans les conditions particulières telles que définies, permet avantageusement de capter l'isobutanal et l'acétone dans le flux à traiter et d'obtenir un flux en sortie ayant des teneurs en isobutanal et en acétone réduites par rapport aux teneurs du flux initial, voire d'éliminer totalement l'isobutanal et l'acétone. En particulier, les teneurs, massiques ou molaires, en isobutanal et en acétone dans l'effluent après traitement selon l'invention peuvent être diminuées d'au moins 90%, préférentiellement au moins 95%, et plus préférentiellement au moins 99% par rapport aux teneurs correspondantes dans la charge oléfinique avant traitement.

L'utilisation d'un tel procédé mais dans des conditions autres que celles définies, en particulier à des températures plus élevées, notamment supérieures à 200°C, pourrait conduire à des capacités d'adsorption plus faibles.

Selon un mode de réalisation préféré de la présente invention, la purification de la charge oléfinique, en particulier l'élimination simultanée de l'isobutanal et de l'acétone, est réalisée dans un réacteur comprenant plusieurs lits fixes placés en parallèle et permutables. Ainsi, il est possible de soustraire un des lits de garde à des fins de régénération lorsque le ou les adsorbants le constituant sont saturés en impuretés, ceci sans arrêt du débit de la charge et donc sans arrêt de production.

Plusieurs options sont envisageables pour la phase de régénération du lit fixe ou lit de garde saturé.

Selon un premier mode de réalisation, le lit de garde saturé peut être extrait du réacteur. Dans ce cas, on peut avantageusement éliminer le liquide par exemple par stripage, puis le solide et nettoyer cette partie du réacteur. Le rechargement peut avantageusement être fait avec une nouvelle charge d'adsorbant, ou bien avec l'ancienne charge d'adsorbant, régénérée au solvant, par exemple par passage d'un solvant entre 30 et 200°C, ou par brûlage, avant de replacer cette partie dans le réacteur.

Selon un deuxième mode de réalisation, la régénération du lit d'adsorbant saturé peut avantageusement se faire par rinçage en ligne avec un solvant à co- ou contre-courant pour désorber les impuretés adsorbées à l'étape d'adsorption. Le solvant est avantageusement un hydrocarbure léger choisi parmi le pentane, l'heptane ou l'hexane. Selon un mode de réalisation de l'invention, le solvant peut être chauffé à une température comprise entre 30 et 350°C. La pression est comprise entre 0,1 et 10 MPa, de préférence comprise entre 0,3 et 5 MPa. Après une séparation en sortie de réacteur des impuretés adsorbées lors de la phase d'adsorption et extraites lors de cette régénération de l'adsorbant, et du solvant de régénération, le solvant est avantageusement recyclé dans le réacteur pour poursuivre la régénération.

Selon un troisième mode de réalisation, la phase de régénération du lit de garde saturé en impuretés est avantageusement réalisée par passage d'un gaz entre 180 et 500°C. Le gaz utilisé peut être un gaz de combustion, de l'air, de l'azote. De préférence le gaz de régénération est l'azote. Selon une variante de l'invention, le gaz de régénération peut contenir de l'eau, entre quelques ppm volumiques et 40% volumique d'eau, de préférence entre 0,1 et 20% volumique et de manière encore plus préférée entre 0,1 et 10% volumique.

De manière préférée, la régénération de l'adsorbant se fait in situ. De manière encore plus préférée, la régénération de l'adsorption se fait in situ avec un gaz chaud.

Dans le cas où la purification selon l'invention est mise en œuvre en lit fixe permutable d'au moins un adsorbant comprenant au moins un matériau à charpente zéolithique, la permutation est avantageusement réalisée lorsque la teneur en impuretés hydrocarbonées oxygénées, dans le mélange de butènes après passage sur l'adsorbant comprenant au moins un matériau à charpente zéolithique est supérieure à 10 ppm massique, de manière préférée supérieure à 5 ppm massique et de manière encore plus préférée supérieure à 1 ppm massique.

De préférence, ledit procédé de purification d'oléfines selon l'invention est utilisé en amont de tout procédé ou toute étape de transformation d'oléfines. Dans un mode de réalisation préféré, ledit procédé de purification peut être placé en amont d'un procédé de métathèse desdites oléfines. Dans le cas où ledit procédé de purification est placé en amont d'un procédé de métathèse, le mélange d'oléfines purifié constitue la charge du procédé de métathèse.

Les exemples qui suivent sont présentés à titre illustratif et non limitatif.

### Description des Figures

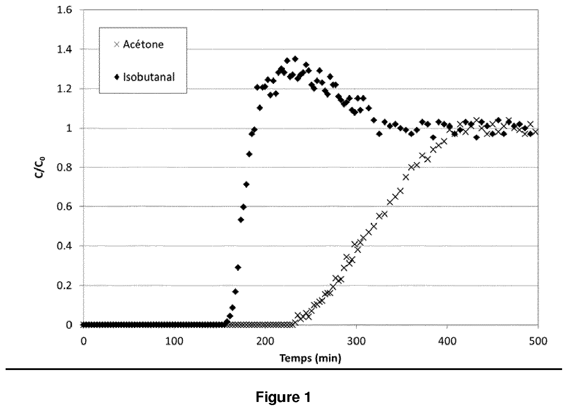

La Figure 1 présente les courbes de perçage de l'acétone et de l'isobutanal obtenues lors de l'essai de co-adsorption sur la zéolithe NaY mise en forme avec de l'argile selon l'exemple A et dans les conditions du test 3 du Tableau 1.

### Exemples

Dans les exemples A, B, C et E ci-dessous, la charge oléfinique a été préalablement débarrassée de son éthanol et de son eau. La charge oléfinique a en effet été lavée à l'eau puis séchée sur un tamis 3A. A l'issue de ce pré-traitement, les teneurs en eau et éthanol de la charge sont vérifiées respectivement par la méthode de Karl Fischer et par chromatographie en phase gazeuse selon la méthode UOP 960. La charge oléfinique qui est testée dans les exemples A, B, C et E ne contient plus ni eau et ni éthanol.

Dans l'exemple D ci-dessous, la charge oléfinique a été préalablement passée sur un tamis 3A. Une analyse par la méthode de Karl Fischer montre qu'à l'issue de ce pré-traitement, la charge oléfinique testée dans l'exemple D ne contient plus d'eau.

### Exemple A. Co-adsorption d'isobutanal et d'acétone et production de butènes purifiés

Une zéolithe NaY de chez Zeolyst mise en forme avec de l'argile, l'argile représentant 15% poids du poids total de l'adsorbant mis en forme, est testée dans un réacteur, sur lit fixe à 30°C sous 0,8 MPa. La zéolithe mise en forme est de couleur blanche. La charge est constituée de 80% de n-butène, de 20% d'isobutène et comprend de l'isobutanal et de l'acétone en concentrations variables (cf. Tableau 1, Tests 1 à 3).

La colonne d'adsorbant est préalablement activée à 350°C sous N₂ pendant 12h puis est remplie de butane à 30°C sous 0,8 MPa. On bascule ensuite sur la charge qui est injectée à un débit de 0,5 ml/min en continu, soit une VVH de 0,5 h⁻¹.

Le suivi de la concentration des différents constituants en sortie de réacteur est réalisé grâce à une analyse chromatographique en phase gaz sur un microGC 4900 de chez Varian. Le test est considéré comme terminé quand la concentration de l'effluent de sortie est la même que celle de la charge en entrée, c'est-à-dire à saturation de l'adsorbant.

A la fin de chaque test, la zéolithe NaY est déchargée. Elle est systématiquement de couleur blanche.

Les capacités de captation de l'adsorbant à saturation en isobutanal et en acétone sont calculées par bilan matière entre ce qui entre et ce qui sort de la colonne. Ces capacités de captation, exprimées en g pour 100 g d'adsorbant, sont reportées dans le Tableau 1 ainsi que les concentrations en isobutanal et acétone de la charge oléfinique, exprimées en ppm massique et leur rapport molaire.

**Tableau1 : Quantités adsorbées en isobutanal et en acétone par la NaY de charges contenant des ratios variables en isobutanal et en acétone**

| Test | [Isobutanal] (ppm massique) | [Acétone] (ppm massique) | Rapport molaire isobutanal/acétone | q_{ads_isobutanal} (g/100g) | q_{ads_acétone} (g/100g) |
|---|---|---|---|---|---|
| 1 | 1922 | 1552 | 1 | 8,2 | 10,4 |
| 2 | 645 | 435 | 1,2 | 9,1 | 9,8 |
| 3 | 638 | 105 | 5 | 8,1 | 3,3 |

Les valeurs de capacités de captation obtenues (q_{ads_isobutanal} , q_{ads_acétone}) montrent que la zéolithe NaY permet de capter de manière importante l'isobutanal et l'acétone présents dans la charge oléfinique de départ, quels que soient les concentrations et le rapport de concentrations en isobutanal et en acétone.

Les courbes de perçage de l'isobutanal et de l'acétone obtenues lors de l'essai de co-adsorption d'isobutanal et d'acétone présents dans la charge à des concentrations respectives de 638 et de 105 ppm massiques, sur la zéolithe NaY mise en forme avec de l'argile (Test 3 selon l'invention du Tableau 1), sont représentées sur la Figure 1.

D'après la Figure 1, il apparait que le procédé selon l'invention, réalisé sur un lit de zéolithe de type FAU (la zéolithe NaY) à 30°C, permet d'obtenir des teneurs en isobutanal et acétone dans l'effluent en sortie de colonne inférieures à 1 ppm massique. En effet, les concentrations en isobutanal et acétone dans l'effluent de sortie de réacteur sont inférieures toutes les deux à la limite de détection de 1 ppm massique, pendant 155 et 230 minutes respectivement pour l'isobutanal et l'acétone.

### Exemple B. Co-adsorption d'isobutanal et d'acétone sur une zéolithe 5A de type LTA (exemple comparatif)

Un test similaire à celui de l'exemple A est réalisé sur une zéolithe 5A de type structural LTA (mise en forme avec 15% poids d'argile) dont les cages ont pour ouverture maximale un anneau constitué de 8 atomes d'oxygène, avec une charge constituée de 80% de n-butène, de 20% d'isobutène, de 647 ppm massique d'isobutanal et de 103 ppm massique d'acétone.

Les capacités de captation exprimées en g pour 100 g d'adsorbant sont reportées dans le Tableau 2 ainsi que les concentrations en isobutanal et acétone de la charge oléfinique exprimées en ppm massique et leur rapport molaire et comparées à celles obtenues dans le test 3 du Tableau 1 de l'exemple A réalisé avec la même charge.

**Tableau 2 : Quantités adsorbées en isobutanal et en acétone par la NaY et par la zéolithe 5A**

| Zéolithe | [Isobutanal] (ppm massique) | [Acétone] (ppm massique) | Rapport molaire isobutanal/acétone | q_{ads_isobutanal} (g/100g) | q_{ads_acétone} (g/100g) |
|---|---|---|---|---|---|
| NaY | 638 | 105 | 5 | 8,1 | 3,3 |
| 5A | 647 | 103 | 5 | 0,6 | 2,1 |

Ces résultats montrent que la zéolithe 5A de type structural LTA dont les cages ont pour ouverture maximale un anneau constitué de 8 atomes d'oxygène n'adsorbe presque pas d'isobutanal. Cette zéolithe de type LTA a des cages d'ouverture trop petite pour laisser entrer l'isobutanal. Il est donc nécessaire d'utiliser des zéolithes dont au moins une ouverture est constitué d'un anneau d'au moins 10 atomes d'oxygène.

### Exemple C. Co-adsorption d'isobutanal et d'acétone sur un adsorbant alumine (exemple comparatif)

Un test similaire à celui de l'exemple A est mené sur une alumine avec une charge constituée de 80% de n-butène, de 20% d'isobutène, de 644 ppm massique d'isobutanal et de 107 ppm massique d'acétone. L'adsorbant alumine est initialement de couleur blanche.

Après le test, l'alumine déchargée est orange. Ceci est dû à une dégradation de l'isobutanal et de l'acétone qui ont réagi ensemble. Cet adsorbant n'est donc pas adapté pour purifier des charges contenant ces deux composés car le produit issu de la réaction entre l'isobutanal et l'acétone est moins bien retenu par l'adsorbant et entraîne donc une perte de capacité de captation de l'adsorbant.

### Exemple D. Co-adsorption d'isobutanal, d'acétone d'une charge contenant également de l'éthanol et production de butènes purifiés (exemple comparatif)

Un test similaire à celui de l'exemple A est mené avec une charge composée de 80% de n-butène, de 20% d'isobutène, et comprenant un mélange quasi équimolaire d'isobutanal (499 ppm molaire soit 642 ppm massique), d'acétone (503 ppm molaire soit 521 ppm massique) et d'éthanol (501 ppm molaire soit 412 ppm massique).

La capacité d'adsorption à saturation de la zéolithe NaY (mise en forme avec 15% poids d'argile), exprimée en g/100g de la zéolithe NaY, en isobutanal, acétone et éthanol est respectivement de 0,9 g/100g, 1,2 g/100g et 17,6 g/100g. La présence d'éthanol en quantité quasi équimolaire par rapport à l'isobutanal et l'acétone entraîne une baisse des capacités d'adsorption de la NaY en isobutanal et en acétone d'un facteur proche de 10. Ces résultats montrent que la présence d'éthanol est très fortement défavorable à la captation d'isobutanal et d'acétone par la NaY.

### Exemple E. Réaénérabilité de l'adsorbant et cyclage

Une zéolithe NaY de chez Zeolyst mise en forme avec 15% d'argile est testée sur lit fixe à 30°C sous 0,8 MPa à un débit de la charge oléfinique de 0,5 ml/min, soit une VVH de 0,5 h⁻¹. La charge est composée de 80% de n-butène, de 20% d'isobutène et comprend 638 ppm massique d'isobutanal et 105 ppm massique d'acétone.

Les concentrations en isobutanal et en acétone dans l'effluent de sortie sont suivies par analyse chromatographique en phase gaz sur un microGC 4900 de chez Varian.

Quand la composition en sortie devient identique à celle en entrée, l'adsorbant est considéré comme saturé. Le débit de la charge est stoppé et l'adsorbant est régénéré sous flux d'azote à pression atmosphérique à 290°C à 20 NL/h. La régénération est considérée comme terminée lorsque les concentrations en isobutanal et en acétone dans le flux de sortie deviennent nulles. Le flux d'azote est alors remplacé par un flux de charge oléfinique, de même débit et même composition que la charge du 1^{er} cycle de manière à procéder à un deuxième cycle d'adsorption de l'isobutanal et de l'acétone. Les concentrations en sortie sont suivies comme précédemment par analyse chromatographique en phase gaz sur un microGC 4900. Quand la composition en sortie devient identique à celle en entrée, la colonne est de nouveau soumise à un flux d'azote à pression atmosphérique à 290°C.

5 cycles d'adsorption/régénération (la dernière régénération n'étant pas effectuée) sont enchainés. Les capacités de captation en isobutanal et en acétone, calculées de la même manière que dans l'exemple A, sont reportées dans le Tableau 3.

**Tableau 3 : Quantités adsorbées en isobutanal et acétone par la zéolithe NaY après chacun des 5 cycles d'adsortion/désortion**

| Cycle | q_{ads_isobutanal} (g/100g) | q_{ads_acétone} (g/100g) |
|---|---|---|
| 1 | 8,1 | 3,3 |
| 2 | 6,0 | 2,8 |
| 3 | 5,1 | 2,7 |
| 4 | 5,4 | 2,7 |
| 5 | 5,2 | 2,7 |

Au bout de cinq cycles d'adsorption et quatre régénérations, les capacités d'adsorption en isobutanal et acétone de la zéolithe NaY testée sont encore satisfaisantes. L'adsorbant NaY est toujours en capacité d'éliminer conjointement l'isobutanal et l'acétone.

## Revendications

1. Procédé de purification d'une charge oléfinique comprenant des oléfines à 4 atomes de carbone et des impuretés incluant l'isobutanal, l'éthanol et l'acétone, ledit procédé comprenant :
a) une étape de pré-traitement comprenant au moins une étape d'élimination de l'éthanol et d'eau ;
b) une étape d'élimination simultanée de l'isobutanal et de l'acétone, par passage de la charge pré-traitée issue de l'étape a) sur au moins un lit fixe d'au moins un adsorbant comprenant au moins un matériau à charpente zéolithique,
ledit matériau à charpente zéolithique étant choisi dans le groupe constitué par: les zéolithes, AIPOs, SAPOs et leurs mélanges ;
ledit matériau à charpente zéolithique possédant au moins des cages ou des canaux dont au moins une ouverture est définie par un anneau à au moins 10 atomes d'oxygène (10MR);
ladite étape b) opérant à une température entre comprise 0 et 200°C, à une pression de 0,1 à 10 MPa et avec une vitesse volumique horaire (VVH) de la charge pré-traitée issue de l'étape a) sur le lit fixe comprise entre 0,1 et 10 h⁻¹.

2. Procédé selon la revendication 1, dans lequel ladite charge oléfinique est issue de la déshydratation de l'isobutanol ou d'un mélange d'isomères du butanol comprenant l'isobutanol.

3. Procédé selon l'une des revendications précédentes, dans lequel l'éthanol est éliminé par lavage à l'eau de la charge oléfinique suivi d'un séchage.

4. Procédé selon l'une quelconque des revendications précédentes, dans lequel la zéolithe est choisie parmi les types FAU, BEA, MOR, MFI, FER, EMT, TON, SZR, LTL.

5. Procédé selon l'une quelconque des revendications précédentes, dans lequel le matériau à charpente zéolithique possède un type structural FAU.

6. Procédé selon l'une quelconque des revendications précédentes, dans lequel le matériau à charpente zéolithique possède un ou plusieurs types de cations compensateurs de charge.

7. Procédé selon la revendication 6, dans lequel le ou les cations compensateurs de charge sont des alcalins.

8. Procédé selon l'une quelconque des revendications précédentes, dans lequel le matériau à charpente zéolithique est mis en forme avec un liant choisi parmi des oxydes ou hydroxydes réfractaires simples ou mixtes, des argiles, des charbons actifs, des résines vinyliques réticulées, et tel que la teneur en liant est comprise entre 5 et 30% en poids de l'adsorbant.

9. Procédé selon l'une quelconque des revendications précédentes, dans lequel l'étape b) opère à une température comprise entre 20 et 60°C.

10. Procédé selon l'une quelconque des revendications précédentes, dans lequel l'étape b) opère à une pression comprise entre 0,3 et 5 MPa.

11. Procédé selon l'une quelconque des revendications précédentes, dans lequel l'étape b) est conduite telle que la vitesse volumique horaire (VVH) de ladite charge sur ledit lit fixe est comprise 0,2 et 5 h⁻¹.

12. Procédé selon l'une quelconque des revendications précédentes, dans lequel l'étape b) est réalisée dans un réacteur comprenant plusieurs lits fixes placés en parallèle et permutables.

13. Procédé de transformation d'oléfines comprenant une étape correspondant au procédé de purification de la charge oléfinique selon l'une des revendications précédentes en amont du procédé de métathèse.

## Patentansprüche

1. Verfahren zur Aufreinigung einer Olefincharge, welche Olefine mit 4 Kohlenstoffatomen sowie Verunreinigungen umfasst, zu denen Isobutanal, Ethanol und Aceton gehören, wobei das Verfahren Folgendes umfasst:
a) einen Schritt der Vorbehandelns, welcher zumindest einen Schritt des Entfernens von Ethanol und Wasser umfasst;
b) einen Schritt des gleichzeitigen Entfernens von Isobutanal und von Aceton, indem die vorbehandelte Charge, welche aus dem Schritt a) stammt, über mindestens ein Festbett aus mindestens einem Adsorptionsmittel geleitet wird, welches mindestens ein Material mit Zeolithgerüst umfasst,
wobei das Material mit Zeolithgerüst aus der Gruppe ausgewählt ist, die aus Folgendem besteht: den Zeolithen, AIPOs, SAPOs und deren Mischungen; wobei das Material mit Zeolithgerüst zumindest Käfige oder Kanäle besitzt, wobei mindestens eine Öffnung derselben durch einen Ring mit mindestens 10 Sauerstoffatomen (10 MR) begrenzt wird;
wobei der Schritt b) bei einer Temperatur im Bereich von 0 bis 200 °C, bei einem Druck von 0,1 bis 10 MPa und mit einem stundenbezogenen Volumendurchsatz (VVH) der vorbehandelte Charge, welche aus dem Schritt a) stammt, durch das Festbett im Bereich von 0,1 bis 10 h⁻¹ betrieben wird.

2. Verfahren nach Anspruch 1, wobei die Olefincharge erhalten wurde, indem Isobutanol oder eine Mischung von Butanolisomeren, welche Isobutanol umfasst, einer Wasserabspaltung unterzogen wurde.

3. Verfahren nach einem der vorhergehenden Ansprüche, wobei der Ethanol entfernt wird, indem die Olefincharge zunächst mit Wasser gewaschen und dann getrocknet wird.

4. Verfahren nach einem beliebigen der vorhergehenden Ansprüche, wobei der Zeolith aus den Typen FAU, BEA, MOR, MFI, FER, EMT, TON, SZR, LTL ausgewählt ist.

5. Verfahren nach einem beliebigen der vorhergehenden Ansprüche, wobei das Material mit Zeolithgerüst eine Struktur des Typs FAU besitzt.

6. Verfahren nach einem beliebigen der vorhergehenden Ansprüche, wobei das Material mit Zeolithgerüst eine oder mehrere Arten von ladungsausgleichenden Kationen besitzt.

7. Verfahren nach Anspruch 6, wobei es sich bei dem oder den ladungsausgleichenden Kationen um Alkalistoffe handelt.

8. Verfahren nach einem beliebigen der vorhergehenden Ansprüche, wobei das Material mit Zeolithgerüst mit einem Bindemittel geformt wird, das aus den einfachen oder gemischten hitzebeständigen Oxiden oder Hydroxiden, den Tonen, den Aktivkohlestoffen, den vernetzten Vinylharzen ausgewählt ist, und zwar derart, dass der Bindemittelgehalt im Bereich von 5 bis 30 Gewichts-% des Adsorptionsmittels liegt.

9. Verfahren nach einem beliebigen der vorhergehenden Ansprüche, wobei der Schritt b) bei einer Temperatur im Bereich von 20 bis 60 °C betrieben wird.

10. Verfahren nach einem beliebigen der vorhergehenden Ansprüche, wobei der Schritt b) bei einem Druck im Bereich von 0,3 bis 5 MPa betrieben wird.

11. Verfahren nach einem beliebigen der vorhergehenden Ansprüche, wobei der Schritt b) derart durchgeführt wird, dass der stundenbezogene Volumendurchsatz (VVH) der Charge durch das Festbett im Bereich von 0,2 bis 5 h⁻¹ liegt.

12. Verfahren nach einem beliebigen der vorhergehenden Ansprüche, wobei der Schritt b) in einem Reaktor durchgeführt wird, der mehrere Festbetten umfasst, welche parallel angeordnet und wahlweise schaltbar sind.

13. Verfahren zur Umwandlung von Olefinen, das im Vorfeld des Metathesenverfahrens einen Schritt umfasst, welcher dem Verfahren zur Aufreinigung der Olefincharge nach einem der vorhergehenden Ansprüche entspricht.

## Claims

1. Process for purifying an olefinic feedstock comprising olefins with 4 carbon atoms and impurities including isobutanal, ethanol and acetone, said process comprising:
a) a pre-treatment step comprising at least a step of eliminating the ethanol and water;
b) a step of simultaneously eliminating the isobutanal and the acetone, by passing the pretreated feedstock from step a) over at least one fixed bed of at least one adsorbent comprising at least one material with a zeolite framework,
said material with a zeolite framework being chosen from the group consisting of: zeolites, AlPOs, SAPOs and mixtures thereof;
said material with a zeolite framework having at least cages or channels of which at least one opening is defined by a ring comprising at least 10 oxygen atoms (10MR);
said step b) being carried out at a temperature of between 0 and 200°C, at a pressure of 0.1 to 10 MPa and with an hourly space velocity (HSV) of the pretreated feedstock from step a) over the fixed bed of between 0.1 and 10 h⁻¹.

2. Process according to Claim 1, wherein said olefinic feedstock results from the dehydration of isobutanol or of a mixture of butanol isomers comprising isobutanol.

3. Process according to either one of the preceding claims, wherein the ethanol is eliminated by washing the olefinic feedstock with water followed by drying.

4. Process according to any one of the preceding claims, wherein the zeolite is chosen from the types FAU, BEA, MOR, MFI, FER, EMT, TON, SZR, LTL.

5. Process according to any one of the preceding claims, wherein the material with a zeolite framework has an FAU structural type.

6. Process according to any one of the preceding claims, wherein the material with a zeolite framework has one or more types of charge-compensating cations.

7. Process according to Claim 6, wherein the charge-compensating cation(s) are alkali metals.

8. Process according to any one of the preceding claims, wherein the material with a zeolite framework is formed with a binder chosen from simple or mixed refractory oxides or hydroxides, clays, active carbons, and crosslinked vinyl resins, and such that the binder content is between 5 and 30% by weight of the adsorbent.

9. Process according to any one of the preceding claims, wherein step b) is carried out at a temperature of between 20 and 60°C.

10. Process according to any one of the preceding claims, wherein step b) is carried out at a pressure of between 0.3 and 5 MPa.

11. Process according to any one of the preceding claims, wherein step b) is carried out such that the hourly space velocity (HSV) of said feedstock on said fixed bed is between 0.2 and 5 h⁻¹.

12. Process according to any one of the preceding claims, wherein step b) is carried out in a reactor comprising several fixed beds which are placed in parallel and which are interchangeable.

13. Process for converting olefins, comprising a step corresponding to the process for purifying the olefinic feedstock according to one of the preceding claims, upstream of the metathesis process.
